Europäisches Patentamt

European Patent Office

Office européen des brevets

(11) Numéro de publication: **0 101 690 B1**

(12) **FASCICULE DE BREVET EUROPEEN**

(45) Date de publication du fascicule du brevet:
30.04.86

(21) Numéro de dépôt: 83900491.8

(22) Date de dépôt: 03.02.83

(86) Numéro de dépôt international:
**PCT/FR 83/00023**

(87) Numéro de publication internationale:
**WO 83/03051 (15.09.83 Gazette 83/21)**

(51) Int. Cl.⁴: **A 61 F 13/00,** A 61 F 13/18

(54) **PROCEDE DE FABRICATION D'ARTICLE A USAGE UNIQUE ET ARTICLE A USAGE UNIQUE DESTINE A ABSORBER LES LIQUIDES.**

(30) Priorité: 02.03.82 FR 8203409

(43) Date de publication de la demande:
07.03.84 Bulletin 84/10

(45) Mention de la délivrance du brevet:
30.04.86 Bulletin 86/18

(84) Etats contractants désignés:
**AT BE CH DE FR GB LI LU NL SE**

(56) Documents cités:
**EP - A - 0 012 295**
**FR - A - 2 246 680**
**FR - A - 2 301 189**
**FR - A - 2 394 287**
**GB - A - 2 055 586**

(73) Titulaire: **BEGHIN-SAY SOCIETE ANONYME,**
**F-59239 Thumeries (FR)**

(72) Inventeur: **HOLVOET, Marcel, Anfreville sur Iton,**
**F-27400 Louviers (FR)**
Inventeur: **MITRANI, Sem, 16, rue des Bergeronnettes,**
**F-91 Ris Orangis (FR)**
Inventeur: **PIGNEUL, Raymond, 2, rue des Vosges,**
**F-68320 Durrenentzen (FR)**

(74) Mandataire: **Quéré, Jean Pierre, BEGHIN-SAY Service**
**Propriété Industrielle 54, Avenue Hoche, F-75008 Paris**
**(FR)**

## Description

La présente invention a pour objet un procédé de fabrication en continu d'un article à usage unique destiné à absorber les liquides, tel que décrit dans le préambule de la revendication 1.

La présente invention concerne également les articles à usage unique obtenus par le procédé selon l'une des revendications 1 à 5, destinés à absorber les liquides, notamment les fluides corporels tels que le sang ou l'urine.

Ces articles peuvent être des pansements, des serviettes périodiques, des couches ou des changes complets pour bébés ou adultes incontinents.

D'une manière générale de tels articles présentent une structure stratifiée formée d'un matelas de fibres cellulosiques permettant d'absorber et de retenir les liquides, placé entre une feuille perméable aux liquides et un film synthétique jouant le rôle de barrière vis à vis des liquides.

La feuille perméable est, le plus souvent, un non-tissé composé de fibres artificielles et/ou synthètiques. Ces dernières années de nombreuses recherches ont porté sur la structure de ce non-tissé: selon le mode de fabrication et la nature des fibres employées, il est possible d'obtenir des non-tissés se laissant rapidement traverser par un liquide, mais conservant très peu d'humidité après absorption du liquide par un matelas de fibres cellulosiques.

Ainsi la surface des articles absorbants, en contact avec la peau d'un utilisateur reste peu mouillée: le confort est amélioré et les risques d'irritations sont sensiblement diminués.

Le film synthétique, barrière aux liquides, est en général un film de polyéthylène gaufré collé au non-tissé le long des bords longitudinaux de l'article : il protège les vêtements ou les sous-vêtements.

Dans certains articles, tels que des changes complets, le film de polyéthylène est replié sur le pourtour de l'article de façon à former une bande étroite hydrophobe et imperméable, du côté de la face en non-tissé : ainsi, lorsque l'article est bien appliqué sur l'utilisateur, les liquides à absorber restent dans la zone délimitée par la bande de polyéthylène. Les risques de fuites sont diminués, mais l'inconvénient majeur est que la peau de l'utilisateur est humide sur toute la surface de l'article.

D'autres articles, tels les serviettes périodiques, sont totalement enveloppés d'un non-tissé et sont pourvus, entre le matelas absorbant et le non-tissé, d'un film synthétique en forme de U protégeant non seulement la face inférieure de l'a rticle mais également les côtés. Récemment, sont apparues sur le marché des serviettes périodiques comportant un film synthétique dont les branches du U sont repliées le long des bords longitudinaux, de façon à former une sorte de gouttière: les risques de fuites latérales sont ainsi pratiquement éliminés mais les extrémités de l'article ne comportent aucune barrière aux liquides.

Pour pallier les inconvénients des articles actuels, la présente invention se rapporte à une structure selon laquelle est placée, entre le non-tissé venant en contact avec la peau d'un utilisateur et la masse absorbante, une feuille imperméable pourvue d'une zone perméable aux liquides, ladite zone étant disposée centralement par rapport à la surface supérieure de la masse absorbante.

Le brevet FR 2301189 décrit déjà une telle structure avec son procédé de réalisation, où la zone perméable aux liquides est réalisée par perforation de la feuille à l'aide d'aiguilles suivie d'un chauffage pour élargir les trous. Le procédé est assez complexe, il ne laisse pas le choix de la forme de l'ouverture et entraîne un gaspillage de matière qui pour des produits fabriqués en très grandes séries n'est pas négligeable.

Selon l'invention, le procédé de fabrication est caractérisé en ce que:

a) une feuille imperméable, avant son dépôt sur les masses absorbantes, passe dans un dispositif la découpant de façon à former une première et une deuxième bandes continues, dans sa zone médiane selon un tracé formé d'une succession de segments de droite, parallèles à l'axe longitudinal de la feuille, lesdits segments de droite:

- étant situés en quinconce alternativement de part et d'autre dudit axe,

- étant décalés l'un par rapport à l'autre, la distance entre deux segments successifs situés d'un même côté de l'axe de la feuille imperméable étant supérieure à la longueur des segments,

et ayant des extrémités reliés par des segments coupant l'axe de la feuille imperméable en leur milieu,

b) la dite première bande est décalée latéralement d'une distance légèrement inférieure à la distance séparant les segments situés de part et d'autre de l'axe de la feuille imperméable,

c) la dite deuxième bande suit un chemin plus long que celui de la première bande, d'une longueur égale au pas de la découpe,

d) les deux bandes sont ensuite déposées sur les masses absorbantes et délimitent entre elles les zones ouvertes perméables aux liquides.

Selon une variante de l'invention les deux bandes sont thermoliées ou collées le long des bords longitudinaux de l'article à un film imperméable continu en constituant la face inférieure.

Selon une variante de l'invention la feuille imperméable est microperforée avant la découpe, par exemple au moyen d'un rouleau à pointes et d'un contre-rouleau en mousse, les deux rouleaux présentant une largeur identique à celle de la feuille.

Cette forme de réalisation permet d'obtenir un article retenant les liquides de façon particuliérement satisfaisante, tout en évitant les phénomènes éventuels de macération.

Selon une autre variante de l'invention, prise ou non en combinaison avec les précédentes, la découpe est assurée par un cuteau traçant une sinusoïde, sur la feuille imperméable en déplacement, l'amplitude de la sinusoïde correspondant à la demi-largeur de la zone perméable et la demi-longueur d'onde étant la longueur de la zone perméable.

L'invention a également pour objet un article à usage unique destiné à absorber les liquides tel que décrit dans la revendication 6.

Un autre film synthétique forme la barrière aux liquides du côté de la face inférieure de l'article. Les extrémités de l'article sont thermoliées ou collées de façon à éviter tout passage de liquide de la masse absorbante vers les faces supérieures et/ou inférieures. La zone permeable est de préférence inférieure à 80% de la surface totale de la masse absorbante.

Grâce à la présente invention, on dispose désormais d'articles à usage unique destinés à l'absorption des liquides, dans lesquels le liquide absorbé est enfermé dans une poche imperméable: les remontées de liquides sur les côtés ou au voisinage des extrémités sont impossibles.

Ces articles sont en général fabriqués à l'aide de machines comportant un dispositif de défibrage de pàte papetière, une section de formation d'un ruban continu de pâte défibrée, un dispositif de découpe dudit ruban en une succession de masses absorbantes séparées.

Selon une forme de l'invention, les bandes de feuilles imperméables sont associées par thermoliage ou collage à un non-tissé avant d'être appliquées sur les masses absorbantes.

D'autres avantages et caractéristiques de l'invention apparaîtront dans la description de plusieurs exemples de réalisation dans lesquels:

- la figure 1 représente une vue de dessus d'un article selon une première forme de l'invention.
- la figure 2 représente une vue de dessus d'un article dont la zone centrale d'absorption est délimitée par deux bandes découpées dans une feuille imperméable.
- la figure 3 est une vue en coupe de la figure 1 selon AA.
- la figure 4 est une vue en coupe de la figure 2 selon AA.
- la figure 5 est une vue en coupe de la figure 2 selon BB.
- la figure 6 est une vue en coupe de la figure 1 selon BB.
- la figure 7 est une vue de bout de la figure 1.
- la figure 8 est une vue de bout de la figure 2.
- la figure 9 est une vue de dessus d'une change complet selon l'invention.
- la figure 10 est une vue en coupe de la figure 9 selon AA.
- la figure 11 est une vue en coupe de la figure 9 selon BB.
- la figure 12 est une vue de bout de la figure 9.

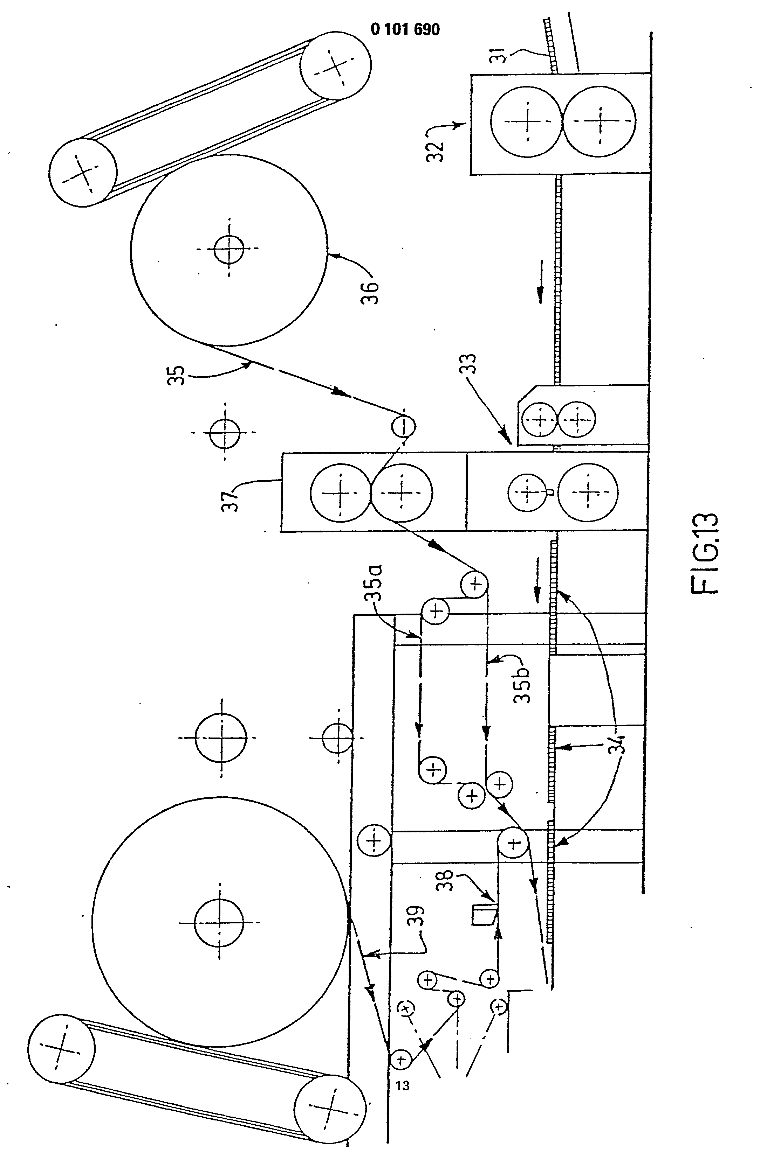

- la figure 13 est un schéma de principe d'une machine servant à fabriquer un article selon l'invention.
- la figure 14 est une vue de dessus d'un film prédécoupé selon un tracé permettant la formation de zones d'absorption.
- la figure 15 est une vue de dessus de deux bandes découpées dans un film selon la figure 14 et associées de façon à former des zones ouvertes.

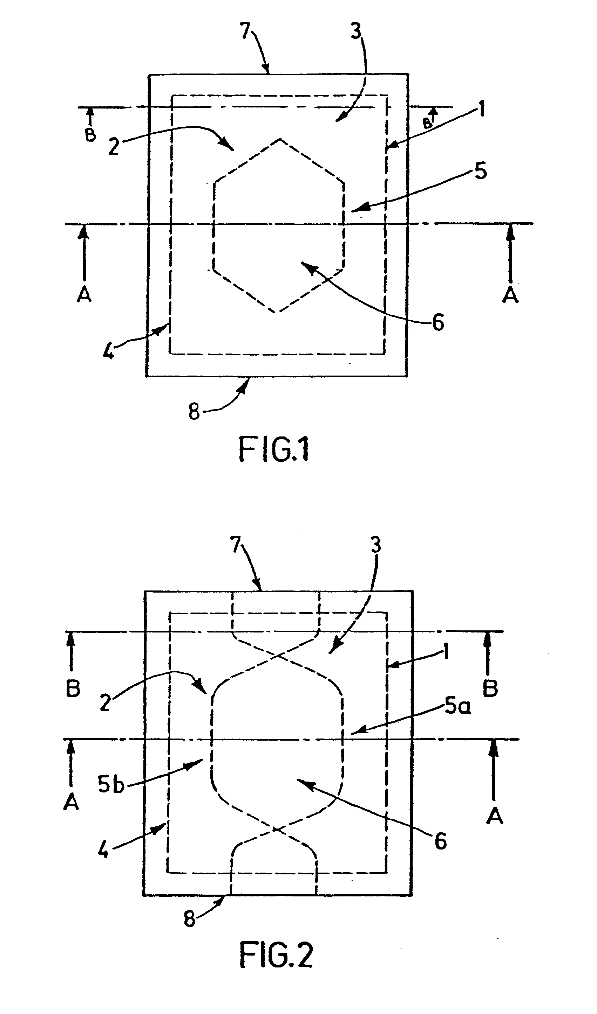

Sur les dessins des figures 1 et 2 est représenté un article selon l'invention, comportant une masse absorbante 1, présentant une face supérieure (2) et une face inférieure (3), constituées d'un nontissé (4) perméable aux liquides. Une feuille imperméable aux liquides (5) est placée entre la masse absorbante (1) et le non-tissé (4) du côté de la face supérieure (2).

Cette feuille (5) est pourvue d'une zone (6) perméable aux liquides et peut être constituée de deux demi-feuilles (5a) et (5b).

L'article présente des extrémités (7) et (8).

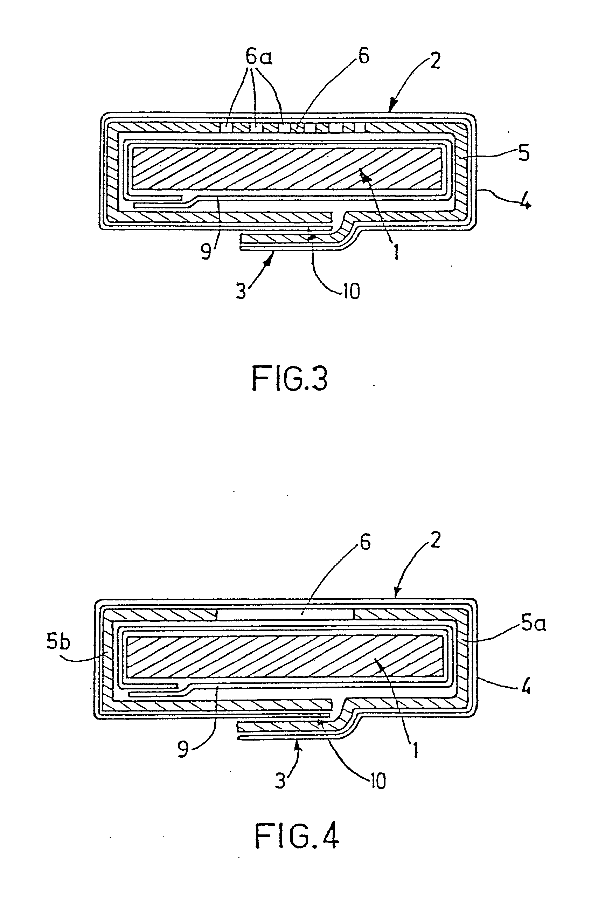

Le dessin de la figure 3 montre une vue en coupe de la figure 1 selon AA: la feuille (5) comporte une zone (6) présentant une multiplicité de perforations (6a). La feuille (5) est revêtue d'un non-tissé (4) et ses bords longitudinaux sont collés ou thermoliés en (10).

Le dessin de la figure 4 montre une vue en coupe de la figure 2 selon AA: la feuille imperméable (5) est constituée de deux demi-feuilles (5a) et (5b) découpées et associées de façon à formerune ouverture oblongue (6).

Les demi-feuilles (5a) et (5b) revetues d'un non-tissé (4) entourent complétement la masse absorbante (1) et sont collées ou thermoliées (en 10) du côté de la face inférieure (3) de façon à ce quela masse absorbante (1) soit complétement enveloppée sauf dans la zone (6).

Avantageusement la masse absorbante (1) est enveloppée dans une feuille de tissue-ouate (9) qui assure une bonne diffusion du liquide. La feuille de tissue-ouate (9) est placée entre la masse absorbante (1) et la feuille imperméable (5a, 5b).

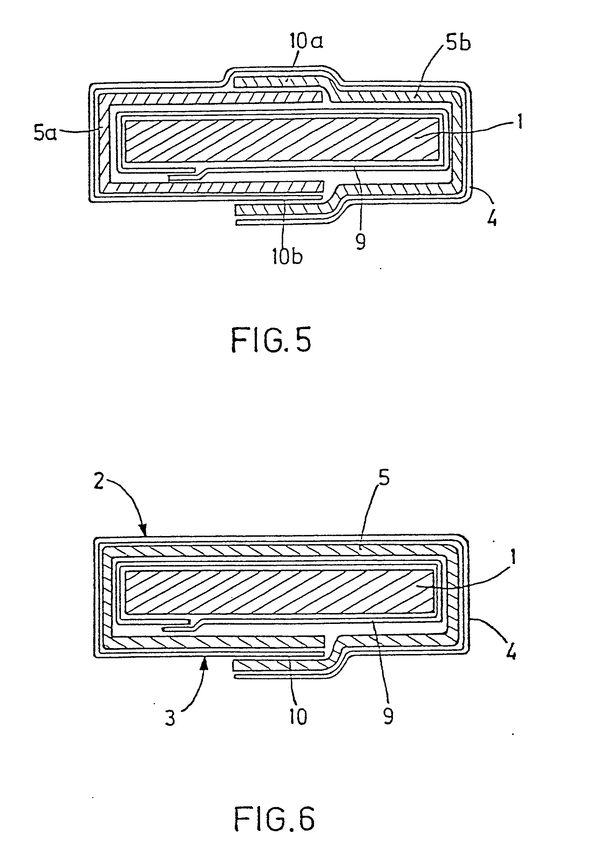

Le dessin de la figure 5 présente une vue en coupe de la figure 2 selon BB: les deux demi-feuilles (5a) et (5b) sont, du côté de la face supérieure (2) comme du côté de la face inférieure (3), collées ou thermoliées de façon à enserrer totalement la masse absorbante (1) au voisinage des extrémités (7) et (8), en (10a) et (10b).

Le dessin de la figure 6 présente une vue en coupe de la figure 1 selon BB : la feuille (5) entoure totalement la masse absorbante (1), et ses bords longitudinaux sont colles ou thermolies du côte de la face inférieure (3) de l'article.

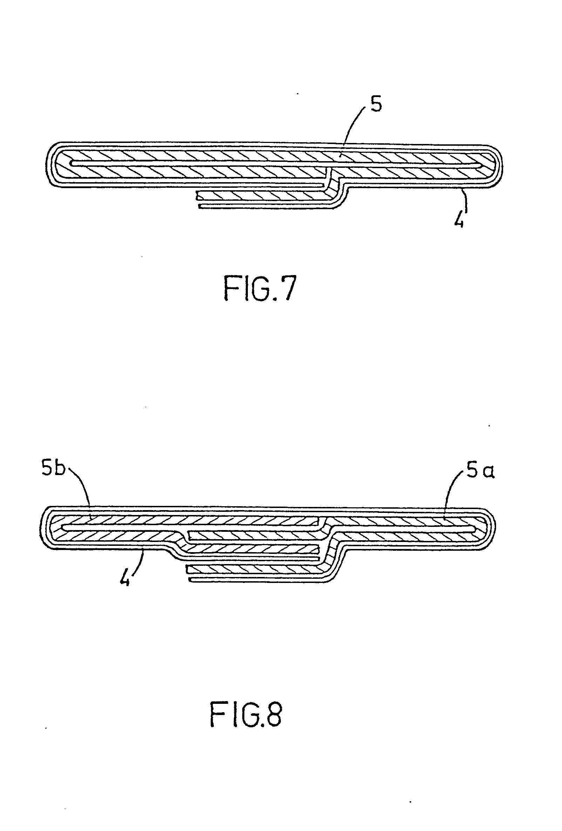

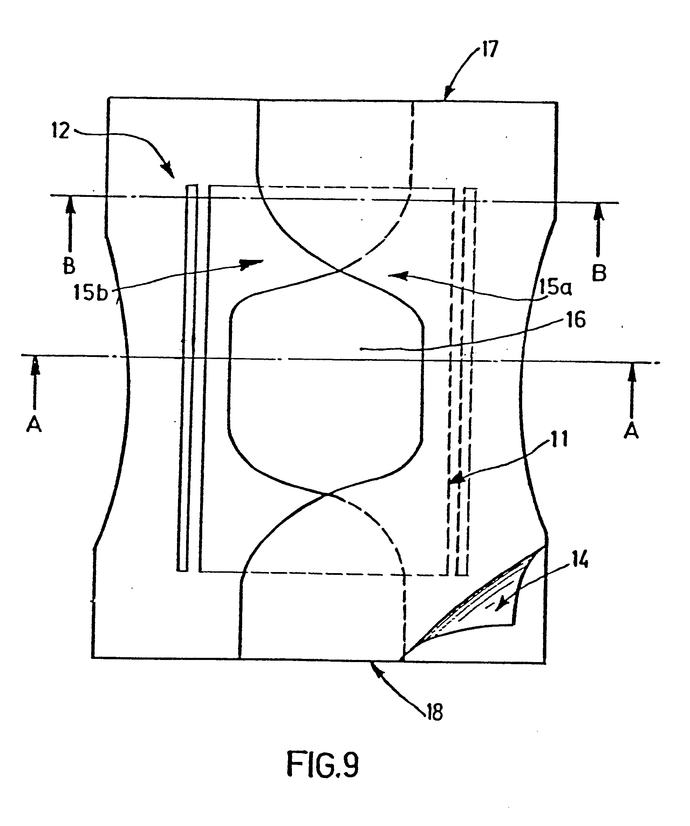

Les dessins des figures 7 et 8 sont des vues de bout des articles représentés par les figures 1 et 2: la feuille (5) - ou les deux demi-feuilles (5a) et (5b) - sont collées ou thermoliées transversalement sur elles-mêmes, de façon à fermer les extrémités (7) et (8)de l'article selon l'invention.

Ainsi, les liquides sont absorbés dans la masse absorbante (1) à travers la zone (6), diffusent dans toute la masse absorbante (1), notamment dans les parties enserrées dans la feuille imperméable (5): les extrémités (7) et (8) étant fermées, les liquides ne peuvent venir remouiller la face supérieure (2) de l'article et le non-tissé (4) ne reste éventuellement humide que dans la zone (6).

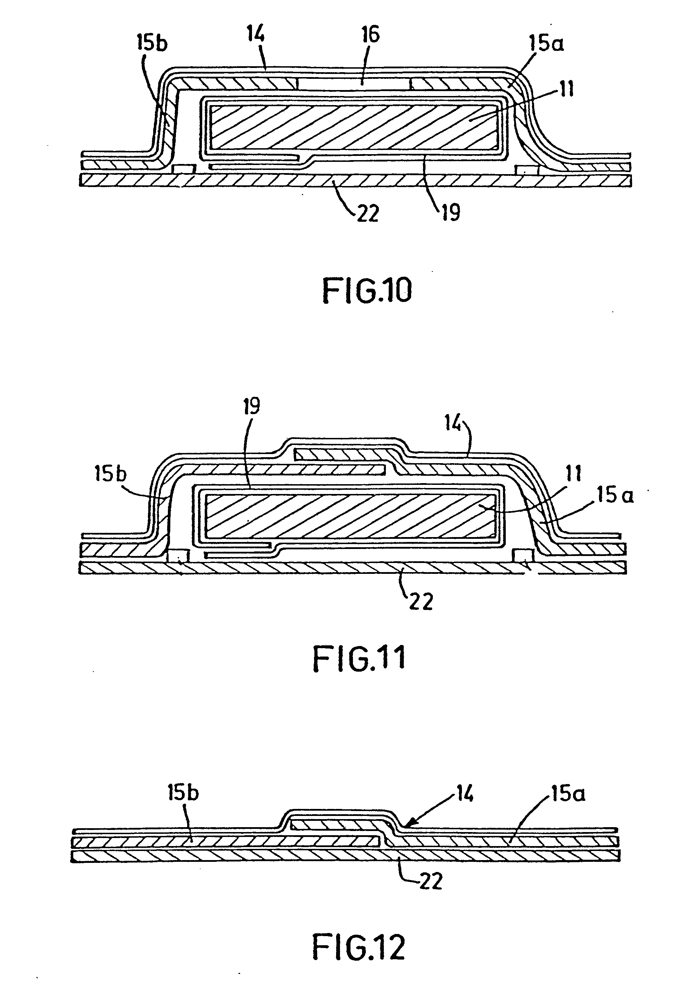

Les dessins des figures 9 à 12 présentent la vue de dessus et des vues en coupe d'un change complet dont la masse absorbante (11), entourée d'une feuille de tissue-ouate (19), est fixée sur un film imperméable (22). Le long des bords longitudinaux de la masse absorbante (11), des laminettes sont collées à l'etat tendu sur le film (22).

Deux bandes (15a) et (15b) découpées dans un film imperméable sont disposées sur la masse absorbante (1 1) et delimitent une zone (16) de passage des liquides.

Au voisinage des extrémités de la masse absorbante (11) les bandes (15a) et (15b) se recouvrent totalement.

Les extrémités (17) et (18) du change complet sont fermées, les films (15a) (15b) et (22) étant collés ou thermolies transversalement.

D'autre part, les bords longitudinaux du change complet sont colles ou thermoliés.

La face supérieure (12) du change complet est constituée d'un non-tissé (14) permeable aux liquides, lequel est collé ou thermolié sur toute sa surface aux deux bandes (15a) et ( 15b).

Le dessin de la figure (13) est un schema de principe du procédé de fabrication d'un articie selon l'invention.

Un ruban de pâte défibrée (31) enveloppe dans une feuille de tissue-ouate passe entre des rouleaux gaufreurs (32) puis dans un dispositif de découpe du ruban (33) en une succession de masses absorbantes séparées (34).

Une feuille imperméable (35) est déroulée d'une bobine mère (36), passe dans un dispositif de découpe (37): on obtient deux demibandes (35a) et (35b).

La bande (35a) suit un chemin plus long que la bande (35b) de façon à former les zones (6) (voir figure 2).

Immédiatement avant d'être appliquées sur les masses absorbantes (34), les bandes (35a) et (35b) sont fixées à l'aide d'une rampe d'encollage (38) au non-tissé (39).

De façon connue en soi, les masses absorbantes (34) séparées sont véhiculées sur un film synthétique destiné à constituer la face arriére du change complet.

On obtient ainsi un fourreau continu constitué d'une succession de masses absorbantes enserrées dans une enveloppe imperméable, sauf dans leur zone centrale supérieure: un dispositif de thermocoupe, par exemple, sépare les articles individuels.

Il est également possible de thermolier transversalement les articles, puis de les séparer à l'aide d'un couteau rotatif.

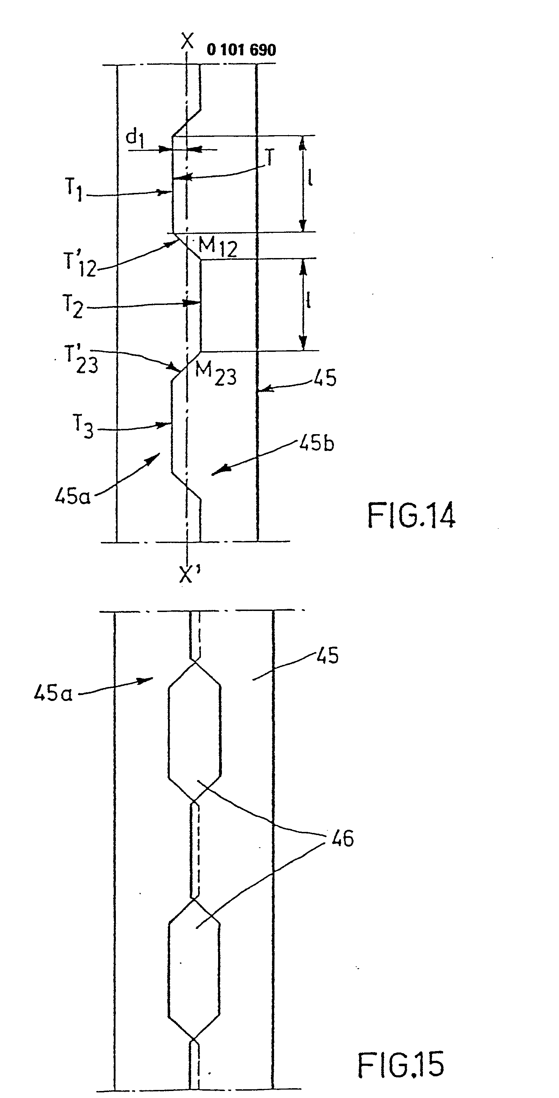

Le dessin des figures 14 et 15 montre comment, à partir d'une feuille (45) on obtient deux demi-bandes (45a) et (45b).

La feuille (45) est découpée selon un tracé (T) formé d'une succession de segments de droite $(T_1, T_2, T_3...)$ de même longueur l, parallélés à l'axe longitudinal XX' de la feuille (45), situés en quinconce alternativement de part et d'autre de XX', décalés l'un par rapport à l'autre, la distance entre deux segments $(T_1)$ et $(T_3)$ situés d'un même côté de XX' étant supérieur à l.

Les extrémités de deux segments $(T_1)$ et $(T_2)$ situés de part et d'autre de XX' sont reliées par un segment $T'_{12}$ dont le milieu de $M_{12}$ se trouve sur l'axe XX,.

La bande (45b) est décalée latéralement d'une distance légèrement supérieure à $d_1$ (distance entre les segments $(T_1)$ $(T_2)$ $(T_3)$ et l'axe XX') puis décalée longitudinalement d'une longueur égaie au pas de la découpe (T) de façon à ce qec deux segments tels que $(T_1)$ et $(T_2)$ deviennent symétriques par rapport à l'axe XX,.

Ainsi, on forme une succession de zones ouvertes (46), les bandes (45a) et (45b) se recouvrant uniquement entre ces zones (46).

A titre d'exemple pour obtenir une zone (46) dont la plus grande dimension est 315 mm et la largeur 110 mm, les dimensions de l'article à fabriquer étant de 550 x 350 mm, on part d'un film de polyéthylène de largeur 300 mm.

On découpe des segments $(T_1)$, $(T_2)$, $(T_3)$..., d'une longueur de 205 mm, parallèles à l'axe XX' et situées à une distance de 30 mm de ce dernier.

Les segments $(T_1)$, $(T_2)$, $(T_3)$ sont disposès de telle sorte que la distance $M_{12} M_{23}$ comprise entre deux segments $(T'_{12})$, $(T'_{23})$ soit de 275 mm.

Le pas de la découpe est de 265 mm.

En décalant latéralement la bande 45b de 50 mm et en la dècalant longitudinalement d'un pas, soit 265 mm, on obtient une succession de zones ouvertes (46) de largeur 110 mm et de longueur maximale 315 mm.

Les deux bandes (45a) et (45b) se recouvrent longitudinalement, au voisinage de l'axe XX', sur 10 mm.

## Revendications

1. Procédé de fabrication en continu d'un article à usage unique destiné à absorber les liquides, à l'aide d'une machine comportant un dispositif de défibrage de pâte papetière, une section de formation d'un ruban continu de pâte défibrée (31), un dispositif de découpe (33) dudit ruban en une succession de masses absorbantes (34), une feuille en matériau imperméable (35; 45) présentant des zones permeables (46) aux liquides étant déroulée à partir d'une bobine mère et déposée sur les masses absorbantes de façon que les zones permeables aux liquides soient situées dans la région centrale des masses absorbantes, les fourreaux obtenus étant sectionnés en articles individuels, ledit procédé est caractérisé en ce que:

a) la feuille imperméable (35, 45) avant son dépôt sur les masses absorbantes, passe dans un dispositif (37) la découpant de façon à former une première et une deuxième bandes continues (35a, 35b; 45a, 45b dans sa zone médiane selon un tracé formé d'une succession de segments de droite ($T_1d$, $T_{2i}$, $T_3$) de même longueur, parallèles à l'axe (XX') longitudinal de la feuille, lesdits segments de droite:

- étant situés en quinconce alternativement de part et d'autre dudit axe,

- étant décalés l'un par rapport à l'autre, la distance entre deux segments successifs situés d'un même côté de l'axe (XX') de la feuille imperméable étant supérieure à la longueur d'un segment,

- ayant des extrémités reliées par des segments ($T'_{12}$ $T'_{23}$) coupant l'axe (XX') de la feuille imperméable en leur milieu ($M_{12}$ $M_{23}$).

b) ladite première bande (35b, 45b) est décalée latéralement d'une distance légèrement inférieure à la distance séparant les segments situés de part et d'autre de l'axe (XX') de la feuille imperméable,

c) ladite deuxième bande (35a, 45a) suit un chemin plus long que celui de la première bande (35b, 45b) d'une longueur égale au pas de la découpe,

d) les deux bandes (35a, 45a, 35b, 45b) sont ensuite déposées sur les masses absorbantes (34) et délimitent entre elles lesdites zones ouvertes (46) perméables aux liquides.

2. Procédé de fabrication en continu d'un article à usage unique selon la revendication 1 caractérisé en ce que les deux (35a, 35b; 45a, 45b) bandes de feuilles imperméables (35, 45) sont associées par thermoliage ou collage à un non-tissé (39) avant d'être déposées sur les masses absorbantes (34).

3. Procédé de fabrication en continu d'un article à usage unique selon la revendication 1, caractérisé en ce que les deux bandes (35a, 35b) sont fixées à l'aide d'une rampe d'encollage (38) au non-tissé (39), immédiatement avant d'être appliquées sur les masses absorbantes (34).

4. Procédé de fabrication en continu d'un article à usage unique selon la revendication 1, caractérisé en ce que le sectionnement du ruban de pâte défibrée (31) enserré entre la feuille imperméable (35, 45) et le film synthétique constituant la face inférieure de l'article est pratiqué dans un dispositif de thermocoupe.

5. Procédé de fabrication en continu d'un article à usage unique selon la revendication 1, caractérisé en ce que les extrémités de chaque article sont thermoliées transversalement avant sectionnement du fourreau en articles individuels.

6. Article à usage unique obtenu par le procédé selon l'une des revendications 1 à 5, destiné à absorber les liquides, comportant une masse absorbante (1,11) dont au moins la face supérieure (2) est constituée d'un non-tissé (4,14) perméable aux liquides, une feuille imperméable (5; 15) pourvue d'une zone centrale (6; 16) perméable aux liquides, découvrant au moins 50 % de la masse absorbante étant placée entre la masse absorbante et le non-tissé caractérisé en ce que la feuille est constituée d'au moins deux bandes (5a, et 5b; 15a, 15b) découpées dans une feuille unique dans le sens longitudinal suivant une ligne brisée ou sinusoïdale permettant après décalage, assemblage la formation de ladite zone perméable (6; 16) aux liquides.

7. Article selon la revendication 6, caractérisé en ce que la feuille imperméable (5,15) est constituée de deux demi-bandes (5a, 5b; 15a, 15b) collées ou thermoliées transversalement sur elles-mêmes de façon à fermer les extrémités (7, 8, 17, 18) de l'article.

8. Article selon la revendication 6, caractérisé en ce que les bandes (5a, 5b) sont collées ou thermoliées du côté de la face inférieure des masses absorbantes.

9. Article selon l'une des revendications 6 ou 7, caractérisé en ce que les bandes (5a, 15a, 5b, 15b) sont thermoliées ou collées longitudinalement avec un film synthétique (22) imperméable aux liquides, constituant la face inférieure (3).

10. Article selon l'une des revendication 6 à 9, caractérisé en ce que la feuille imperméable (5) est collée ou thermoliée au non-tissé (4) sur toute la surface de la face supérieure (2).

11. Article selon l'une des revendications 6 à 1 0, caractérisé en ce qu'il est un pansement.

12. Article selon l'une des revendications 6 à 10, caractérisé en ce qu'il est une serviette périodique.

13. Article selon l'une quelconque des revendications 6 à 10, caractérisé en ce qu'il est une couche pour bébé ou pour adulte incontinent.

14. Article selon l'une quelconque des revendications 6 à 10, caractérisé en ce qu'il est un change complet pour bébé ou pour adulte incontinent.

15. Article selon la revendication 14, caractérisé en ce qu'il comporte des moyens élastiques disposés dans la zone de l'entrejambe.

**Claims:**

1. Process for the continuous production of a disposable article intended for absorbing liquids, by means of a machine comprising a paper-pulp defibration device, a section for forming a continuous strip of defibrated pulp (31), and a device (33) for cutting the said strip into a succession of absorbent masses (34), a sheet of impermeable material (35, 45) which has zones (46) permeable to liquids being unwound from a master reel (36) and laid on the absorbent masses, so that the zones permeable to liquids are located in the central region of the absorbent masses, the sheaths obtained being severed into individual articles, the said process being characterised in that:

a) the impermeable sheet (35, 45), before being laid on the absorbent masses, passes into a device (37) which cuts it in its central zone into a first and a second continuous band (35a, 35b; 45a, 45b) along a line formed by a series of straight segments (T1, T2, T3) of the same length and parallel to the longitudinal axis (XX') of the sheet, the said straight segments being arranged staggered alternately on either side of the said axis, being offset relative to one another, with the distance between two successive segments located on one and the same side of the axis (XX') of the impermeable sheet being greater than the length of a segment, and having ends connected by segments (T'12, T'23) intersecting the axis (XX') of the impermeable sheet at their centre (M12, M23),

b) the said first band (35b; 45b) is offset laterally by a distance slightly less than the distance separating the segments located on either side of the axis (XX') of the impermeable sheet,

c) the said second band (35a; 45a) follows a path which is longer than that followed by the first band (35b; 45b) by a length equal to the cutting pitch, and

d) the two bands (35a, 45a and 35b, 45b) are subsequently laid on the absorbent masses (34) and delimit between them the said open zones (46) permeable to liquids.

2. Process for the continuous production of a disposable article according to Claim 1, characterised in that the two bands (35a, 35b, 45a, 45b) of impermeable sheets (35, 45) are joined to a non-woven (39) by means of thermal bonding or gluing, before being laid on the absorbent masses (34).

3. Process for the continuous production of a disposable article according to Claim 1, characterised in that the two bands (35a, 35b) are secured to the non-woven (39) by means of a glue-coating rack (38), immediately before being laid on the absorbent masses (34).

4. Process for the continuous production of a disposable article according to Claim 1, characterised in that the strip of defibrated pulp (31) gripped between the impermeable sheet (35, 45) and the synthetic film constituting the lower face of the article is severed in a thermocutting device.

5. Process for the continuous production of a disposable article according to Claim 1, characterised in that the ends of each article are thermally bonded transversely before the sheath is severed into individual articles.

6. Disposable article obtained by means of the process according to one of Claims 1 to 5 and intended for absorbing liquids, incorporating an absorbent mass (1; 11), of which at least the upper face (2) consists of a non-woven (4; 14) permeable to liquids, an impermeable sheet (5; 15) provided with a central zone (6; 16) permeable to liquids, exposing at least 50% of the absorbent mass, being placed between the absorbent mass and the non-woven, characterised in that the sheet consists of at least two bands (5a, 5b; 15a, 15b) cut from a single sheet in the longitudinal direction along a broken or sinusoidal line, making it possible, after they have been offset relative to one another and joined together, to form the said zone (6; 16) permeable to liquids.

7. Article according to Claim 6, characterised in that the impermeable sheet (5; 15) consists of two half-bands (5a, 5b; 15a, 15b) glued or thermally bonded transversely on themselves so as to close the ends (7, 8; 17, 18) of the article.

8. Article according to Claim 6, characterised in that the bands (5a, 5b) are glued or thermally bonded on the same side as the lower face of the absorbent masses.

9. Article according to one of Claims 6 or 7, characterised in that the bands (5a, 15a; 5b, 15b) are thermally bonded or glued longitudinally to a synthetic film (22) impermeable to liquids and constituting the lower face (3).

10. Article according to one of Claims 6 to 9, characterised in that the impermeable sheet (5) is glued or thermally bonded to the non-woven (4) over the entire surface of the upper face (2).

11. Article according to one of Claims 6 to 10, characterised in that it is a dressing.

12. Article according to one of Claims 6 to 10, characterised in that it is a sanitary towel.

13. Article according to any one of Claims 6 to 10, characterised in that it is a napkin for a baby or an incontinent adult.

14. Article according to any one of Claims 6 to 10, characterised in that it is a complete change for a baby or an incontinent adult.

15. Article according to Claim 14, characterised in that it incorporates elastic means arranged in the zone of the crutch.

**Patentansprüche**

1. Verfahren zur kontinuierlichen Herstellung eines Gegenstandes für einen einmaligen Gebrauch zum Aufsaugen von Flüssigkeiten, mit Hilfe einer Maschine bestehend aus einer Vorrichtung zur Entfaserung von Papierpaste, einem Abschnitt zur Bildung eines kontinuierlichen Bandes aus der entfaserten Paste (31), einer Vorrichtung zum Aufschneiden (33) des Bandes in eine Aufeinanderfolge von absorbierenden Massen (34), wobei eine flüssigkeitsdurchlässige Zonen (46) aufweisende Folie aus undurchlässigem Material (34, 35) von einer Mutterspule (36) abgerollt und auf die absorbierenden Massen derart aufgelegt wird, daß die flüssigkeitsdurchlässigen Zonen in dem mittleren Bereich der absorbierenden Massen liegen, und die erhaltenen Hülen in einzelne Gegenstände unterteilt werden, dadurch gekennzeichnet, daß

a) die undurchlässige Folie (35, 45) vor ihrer Auflagerung auf die absorbierenden Massen eine Vorrichtung (37) passiert,welche sie derart aufschneidet, daß in ihrer mittleren Zone ein erstes und ein zweites kontinuierliches Band (35a, 35b; 45a, 45b) gemäß einer Markierung gebildet werden, die aus einer Aufeinanderfolge von geraden Segmenten(T1' T2' T) gleicher Länge und parallel zur Längsachse (XX') der Folie gebildet ist, wobei die genannten geraden Segmente

- in einer Zickzackform abwechselnd auf der einen und der anderen Seite der genannten Achse liegen,

- mit Bezug aufeinander versetzt sind und der Abstand zwischen zwei aufeinanderfolgenden, auf derselben Seite der Achse (XX') der undurchlässigen Folie liegenden Segmente größer ist als die Länge eines Segmentes,

- durch die Achse (XX') der undurchlässigen Folie in ihrer Mitte (M, M) schneidende Segmente (T' 2' T'23) verbundene Enden aufweisen,

b) das genannte erste Band (35b; 45b) seitlich um eine Entfernung versetzt ist, die etwas geringer ist als der Abstand, welcher die auf beiden Seiten der Achse (XX') der undurchlässigen Folie gelegenen Segmente trennt,

c) das genannte zweite Band (35a; 45a) einem Weg folgt, der um eine Länge gleich einem Schritt des Schneidvorgangs länger ist als derjenige des ersten Bandes (35b; 45b),

d) die zwei Bänder (35a, 45a; 35b, 45b) anschließend auf die absorbierenden Massen (34) aufgelegt werden und zwischen sich die genannen flüssigkeitsdurchlässigen offenen Zonen (46) begrenzen.

2. Verfahren zur kontinuierlichen Herstellung eines Gegenstandes für einen einmaligen Gebrauch nach Anspruch 1, dadurch gekennzeichnet, daß die zwei Bänder (35a, 35b; 45a, 45b) der undurchlässigen Folien (35, 45) vor dem Auflegen auf die absorbierenden Massen (34) durch eine Warmverbindung oder durch Kleben mit einem Nichtgewebe (39) verbunden werden.

3. Verfahren zur kontinuierlichen Herstellung eines Gegenstandes für einen einmaligen Gebrauch nach Anspruch I, dadurch gekennzeichnet, daß die zwei Bänder (35a, 35b) unmittelbar vor dem Aufbringen auf die absorbierenden Massen (34) mit Hilfe einer Kleberampe (38) auf dem Nichtgewebe (39) fixiert werden.

4. Verfahren zur kontinuierlichen Herstellung eines Gegenstandes für einen einmaligen Gebrauch nach Anspruch 1, dadurch gekennzeichnet, daß das Aufschneiden des Bandes aus der entfaserten Paste (31), welches zwischen der undurchlässigen Folie (35, 45) und dem die Unterseite des Gegenstandes bildenden synthetischen Film eingeschlossen ist, in einer Wärmeschneidvorrichtung durchgeführt wird.

5. Verfahren zur kontinuierlichen Herstellung eines Gegenstandes für einen einmaligen Gebrauch nach Anspruch 1, dadurch gekennzeichnet, daß die Enden jedes Gegenstandes vor dem Aufschneiden der Hülle in einzelne Gegenstände in Querrichtung durch Wärme verbunden werden.

6. Durch das Verfahren nach einem der Ansprüche 1 bis 5 erhaltener Gegenstand für einen einmaligen Gebrauch zur Aufnahme von Flüssigkeiten, bestehend aus einer absorbierenden Masse (1, 11), deren Oberseite (2) wenigstens aus einem flüssigkeitsdurchlässigen Nichtgewebe (4, 14) besteht, wobei eine mit einer flüssigkeitsdurchlässigen mittleren Zone (6, 16) versehene undurchlässige Folie (5, 15), die wenigstens 50% der absorbierenden Masse bedeckt, zwischen der absorbierenden Masse und dem Nichtgewebe angeordnet ist, dadurch gekennzeichnet, daß die Folie aus wenigstens zwei Bändern (5a, 5b; 15a, 15b) besteht, die aus einer einzigen Folie in Längsrichtung nach einer unterbrochenen oder sinusförmigen Linie geschnitten sind, die nach dem Versetzen und Zusammenfügen die Bildung der genannten flüssigkeitsdurchlässigen Zone (6, 16) gestattet.

7. Gegenstand nach Anspruch 6, dadurch gekennzeichnet, daß die undurchlässige Folie (5, 15) durch zwei Bandhälften (5a, 5b, 15a, 15b) gebildet ist, die auf sich selbst derart in Querrichtung verklebt oder durch Wärme verbunden sind, daß sie die Enden (7, 8; 17, 18) des Gegenstandes schließen.

8. Gegenstand nach Anspruch 6, dadurch gekennzeichnet, daß die Bänder (5a, 5b) auf der Unterseite der absorbierenden Massen verklebt oder durch Wärme verbunden sind.

9. Gegenstand nach einem der Ansprüche 6 oder 7, dadurch gekennzeichnet, daß die Bänder (5a, 15a; 5b, 15b) in Längsrichtung mit einem gegenüber Flüssigkeiten undurchlässigen synthetischen Film (22) durch Wärme verbunden oder verklebt sind, der die Unterseite (3) bildet.

10. Gegenstand nach einem der Ansprüche 6 bis 9, dadurch gekennzeichnet, daß die undurchlässige Folie (5) auf der gesamten Fläche der Oberseite 2 mit dem Nichtgewebe (4) verklebt oder durch Wärme verbunden ist.

11. Gegenstand nach einem der Ansprüche 6 bis 10, dadurch gekennzeichnet, daß er einen Verband darstellt.

12. Gegenstand nach einem der Ansprüche 6 bis 10, dadurch gekennzeichnet, daß er eine Damenbinde darstellt.

13. Gegenstand nach irgendeinem der Ansprüche 6 bis 10, dadurch gekennzeichnet, daß er eine Windel für Babies oder inkontinenzkranke Erwachsene darstellt.

14. Gegenstand nach irgendeinem der Ansprüche 6 bis 10, dadurch gekennzeichnet, daß er eine vollständige Wechselgarnitur für Babies oder inkontinenzkranke Erwachsene darstellt.

15. Gegenstand nach Anspruch 14, dadurch gekennzeichnet, daß er in der Schrittzone angeordnete Elastikeinrichtungen enthält.

FIG.1

FIG.2

# FIG.3

# FIG.4

FIG.5

FIG.6

FIG.7

FIG.8

FIG.9

FIG.10

FIG.11

FIG.12

FIG.13

0 101 690

0 101 690

FIG.14

FIG.15

15